Europäisches Patentamt

European Patent Office

Office européen des brevets

(11) Publication number: **0 600 087 A1**

## EUROPEAN PATENT APPLICATION
### published in accordance with Art. 158(3) EPC

(21) Application number: 92918024.8

(22) Date of filing: **19.08.92**

(86) International application number:
**PCT/JP92/01045**

(87) International publication number:
**WO 93/04369 (04.03.93 93/06)**

(51) Int. Cl.⁵: **G01N 33/15**

(30) Priority: **19.08.91 JP 206956/91**

(43) Date of publication of application:
**08.06.94 Bulletin 94/23**

(84) Designated Contracting States:
**DE FR GB IT**

(71) Applicant: **SHISEIDO COMPANY LIMITED**
**7-5-5 Ginza**
**Chuo-ku Tokyo 104(JP)**

(72) Inventor: **HAYASHI, Toshikatsu, The Shiseido**
**Research Center**
**1050, Nippa-cho,**
**Kohoku-ku**
**Yokohama-shi, Kanagawa 223(JP)**
Inventor: **ITAGAKI, Hiroshi, The Shiseido**
**Research Center**

**1050, Nippa-cho,**
**Kohoku-ku**
**Yokohama-shi, Kanagawa 223(JP)**
Inventor: **TAMURA, Uhei, The Shiseido**
**Research Center**
**1050, Nippa-cho,**
**Kohoku-ku**
**Yokohama-shi, Kanagawa 223(JP)**
Inventor: **KATO, Shinobu, The Shiseido**
**Research Center**
**1050, Nippa-cho,**
**Kohoku-ku**
**Yokohama-shi, Kanagawa 223(JP)**

(74) Representative: **Rinuy, Santarelli**
**14, avenue de la Grande Armée**
**F-75017 Paris (FR)**

(54) **METHOD AND KIT OF IN VITRO IRRITATION TEST.**

(57) This invention provides a method of in vitro testing for evaluating a degree of stimulation imposed on an organism by an alien substance. The invention provides an in vitro stimulation testing method comprising such steps that, in aqueous solution, an alien substance (substance to be tested) is brought into contact with protein having absorption spectrum at its visible part, variation in absorption spectrum resulting from said contact is measured, and then an amount of said variation is evaluated as an index of stimulation on the organism caused by the substance to be tested. Further provided by this invention is a method of testing an organism stimulation by an alien substance which employs a combination of the abovesaid method with other in vitro stimulation testing methods. A kit for performing such testing methods is also provided. The methods of this invention show a satisfactory correlation with the Draze method as an in vivo testing method.

EP 0 600 087 A1

TECHNICAL FIELD

The present invention relates to an in vitro method for testing irritation of the living body based on a test substance and a kit for that test method. According to the present invention, the irritation of the body due to a foreign substance can be predicted by the change in the absorption spectrum derived from denaturation of a particular protein. Furthermore, by incorporating into the above assay system the results of a test method using destruction of liposomes, erythrocytes, and the like, other than the particular protein, as indicators, it is possible to enhance the correlation with results from an in vivo irritation test, in particular, a Draize eye irritation test.

BACKGROUND ART

The assay and evaluation as to whether or not a foreign substance gives harmful irritation to a living body which induces vital reactions, for example, inflammation, hemolysis, opacification, and swelling, are essential in the product development in the technical fields of cosmetics, medicines, and other medical materials and oleo chemicals where a particular substance is directly applied to the living body At the present time, experimental animals are mainly used for these assay and evaluation. In the tests, particularly in irritation tests, use is made of a skin irritation test (primary irritation and accumulating irritation), an ocular irritation test, a patch test, and a mucous membrane irritation test (tunica mucosa oris and urogenital mucosa membrane). For example, in the ocular irritation test, the Draize method, which uses a rabbit, is generally adopted. In this method, the ocular irritation by a test substance is assayed particularly through (1) the determination of the degree of clouding of the cornea, (2) the evaluation of the incidence of congestion or edema of the conjunctiva, and (3) the evaluation of the incidence of congestion or swelling of the iris.

However, the recent rising movement against animal tests from the viewpoint of the prevention of cruelty to animals has led to spirited development of alternative tests not using animals (in vitro test).

As a result, for example, "EYTEX" (trademark, an ocular irritation test system developed by Ropak Laboratories, U.S.A., wherein, a reaction similar to clouding of the cornea caused by a test substance is reproduced in a cuvette. This kit uses clouding of a protein solution caused by a test substance as an indicator), "SKINTEX" (trademark, an alternative test method for skin irritation, same company), and "SOLATEX" (trademark, an alternative test method for phytotoxicity, same company) have become commercially available. Examples of other test kits include alternative test kits for artificial skin "TESTSKIN" (trademark Organogenesis, U.S.A.) and "SKIN2" (trademark, Malotex, U.S.A.). In all of these kits, collagen is contracted by human fibroblasts (dermal model) and, further, human keratinocytes are overlaid (cortex model). Detail, on "EYTEX" etc. are disclosed in R.J. Sato, et al., Toxic. in Vitro Vol. 4, pp.332-335 (1990); V.C. Gordon et al., ibid. pp. 314-417 (1990) and IN VITRO TOXICOLOGY, Approaches to Validation (published, pp.293-301, 1987, Mary Ann Liebert, Inc., 3rd Avenue, New York, NY 10128). The dermal model and the cortex model are used for an alternative method for ocular irritation and an alternative method for skin irritation, respectively.) and a kit wherein use is made of cultured cells "EPIPACK" (trademark, Cornetics, U.S.A., cultured cell kit wherein use is made of keratinocytes). Additionally, there are described in the J. Soc. Cosnut. Chem. Japan Vol. 23, No. 4, pp.272-277 (1990), a red blood cell hemolysis test method (hereinafter abbreviated as the "RBC method"), and toxicity test method using rabbit corneal cells (hereinafter abbreviated as "the SIRC method"). In Japanese Unexamined Patent Publication (Kokai) No. 63-228060, there is disclosed method using disruption of liposomes as an indication (hereinafter abbreviated as "the LIPOSOME method").

Although all the above-described procedures exhibit a certain correlation with respective in vivo test results to such an extent that the predetermined object can be attained, the correlation and procedure are not always satisfactory. For example, a detailed description will now be given on a test method described in K. Miyazawa et al., International Journal of Cosmetic Science, 6: pp. 33-46 (1984), wherein use is made of albumin and denaturation of protein is used as an indicator. In this method, the action of a test substance against the living body is assayed by isolating albumin, which has been allowed to coexist with a test substance (for example, a surfactant), from the test substance by liquid chromatography through the utilization of a peak of an absorption spectrum in an ultraviolet region (at 220 nm) attributable to albumin which is a protein related to a living body, and quantitatively determining the isolated albumin by means of an ultraviolet spectrophotometer to detect the degree of denaturation of albumin. In recent years, this method has become utilized widely in the cosmetic industry and fat chemical industry. It, however, has drawbacks including the fact that liquid chromatography is time-consuming, it is difficult to use this method in an opaque system (an emulsion system or a formulation system wherein a pearling agent is incorporated), and a surfactant or the like often exhibits an interfering peak.

Accordingly, an object of the present invention is to provide an in vitro irritation test method wherein the irritation of a living body based on a foreign substance (a test substance) is assayed by taking advantage of the denaturation of a protein, which test method can be practiced according to a simpler procedure and has a good correlation with in vivo tests as compared with the prior art.

DISCLOSURE OF THE INVENTION

The present inventors have made studies on a correlation of denaturation of various proteins with irritation of a living body with a view to attaining the above-described object and, as a result, have surprisingly found that the ratio of the denaturation caused through contact of a metalloprotein or a modified protein having an absorption spectrum in a visible region with a test substance has a good correlation with irritation of a living body, in particular, the Draize score, which has led to the completion of the present invention.

Accordingly, the above-described problems can be solved by an in vitro of irritation test for assaying irritation of a living body based on a test substance, comprising the steps of:

(A) bringing a test substance into contact with a metalloprotein or a modified protein having an absorption spectrum in a visible region in an aqueous solution;

(B) determining the change in the absorption spectrum attributable to that contact; and

(C) assaying the degree of change in the spectrum as an indicator of the irritation of the living body based on the test substance.

Since the test results have a good correlation with those of the Draize test, in order to elucidate the effective factors which mainly effect the test results, a variety of surfactants were evaluated by the Known in vitro test methods for irritation including these, for example, the "EYTEX", "SIRC", "RBC", and "LIPOSOME" methods, as well as a cytotoxic test using culured cells derived from human cervical carcinoma (hereinafter abbreviated as the "Hela method"; see H. Itagaki et al., Toxic in Vitro Vol. 5, pp.301-304 (1991)) and a toxicity test using chorioallantois of ovum gallinaceum containing sperm (hereinafter abbreviated as the "CAM method"; see S. Hagino et al., Toxic. in Vitro Vol. 5, pp.339-343 (1991)). The data thus obtained are subjected to a factor analysis, (primary factor analysis and standard Varimax rotating operation). As a result, it was found that the Draize test is a test method which observes the composite action of membrane disrupting factors and protein denaturation factors and that the protein disrupting factors are predominant. The test method of the present invention observes mainly the results of action of the protein denaturation factors, so it was guessed that there was a good correlation with Draize test.

As described above, it is believed that in the Draize test, there are included results of action of membrane disrupting factors, though only secondarily, so there is an interest in a series of test systems evaluate the results of an in vitro irritation test method believed to observe the action of membrane disrupting factors by combination with the test results of the present invention. The inventors made a general evaluation by combining the results of test method of the present invention on a single test substance and the results of other test methods displaying good correlation with, in particular, the Draize test, whereupon they confirmed that a higher correlation with the results of the Draize test can be obtained compared with when the former is performed alone and, thereby completed the second invention.

According to the present invention, there is provided an in vitro test method of irritation of a living body based on a test substance, comprising the steps of:

a) a step of conducting separately the test method of first invention and at least one other in vitro irritation test which demonstrates a certain correlation with the Draize eye irritation test with respect to a single test substance and

b) a step of evaluating collectively the results based on the step a).

Furthermore, the above-described object can be attained by providing a kit for an in vitro test method for irritation of a living body based on a test substance, comprising:

a) a device for bringing a test substance into contact with a metalloprotein or a modified protein having an absorption spectrum in a visible region in an aqueous solution and the protein and

b) means for assaying the change in the absorption spectrum caused in the device as an indicator of the irritation of the living body based on the test substance,

wherein the kit is used for carrying out the first invention, and a kit comprising further the test material, required to conduct the second invention.

BEST MODE FOR CARRYING OUT THE INVENTION

The term "living body" used herein means living mammals and human beings, and the term "irritation" means an action which gives rise to some response in the living body particularly through the skin and eye as viewed from the standpoint of sites and the skin and mucous membranes as viewed from the standpoint of physiology and anatomy. In general, skin irritation results in the occurrence of erythema, edema, necrosis, crusting, and desquamation, ocular irritation results in clouding in the cornea and abnormal crease, congestion, swelling, and hyperemia in the iris, and mucosa membrane irritation results in fading, necrosis, ulceration, fissure, and bleeding.

Therefore, with respect to irritation of living bodies by test substances, the method of the present invention can assay the irritation associated with at least one of the above-described symptoms caused in the living bodies. Among them, the irritation to which the method of the present invention can be conveniently applied is irritation which can be assayed by the above-described Draize method.

The term "in vitro irritation test" is the counterpart of an in vivo test such as the above-described Draize method and is for assaying a particular type of irritation without use of any living body per se, so usually is called an alternative method for in vivo tests.

The protein having an absorption spectrum in a visible region used in the first invention means a protein which has a color visible with the naked eye or a light absorption spectrum in a wavelength range from from about 360 to 830 nm in the form of aqueous solution and can be used for the purpose of the present invention. Specific examples thereof include metalloproteins, such as hemoglobins, cytochrome, myoglobin, adrenodoxin, plastocyanin, alcohol dehydrogenase, carbonic anhydrase, carboxylase, phosphohydrase, phosphotransferase, arginase, hemerythrin, ferredoxin, pyrocatechase, peroxidase, catalase, transferrin, ferritin, ascorbate oxidase, cytosidase, cytochrome oxidase, pyruvate kinase, nitrogenase and nitrate reductase, and modified proteins modified with a dye or a fluorescent substance, for example, bovine serum albumin to which tetraethylrhodamine B isothiocyanate or texas red has been bonded. Examples of proteins particularly useful from the viewpoint of easiness of producing a preparation having stable properties and easiness of detecting the modification of the protein through a change in the color include hemoglobin and its affinitive substances (or analogs), cytochrome C and its analogs, adrenodoxin, and plastocyanin.

These proteins are selected depending upon the properties of the test substance. For example, proteins having a relatively low isoelectric point, that is, adrenodoxin, plastocyanin, and cytochromes other than cytochrome C, are selected for the assay of an acidic test substance, cytochrome C is selected for the assay of an alkaline test substance, and hemoglobin is used for the assay of a neutral test substance. Hemoglobin is usually employed.

The aqueous solution for dissolving the above-described proteins is preferably buffered with a suitable buffer. Further, it may contain organic solvents miscible with water, for example, lower alcohols (such as methanol, ethanol and isopropanol), acetone, diglyme, tetrahydrofuran, dioxane, dimethylformamide, and dimethylsulfoxide.

When hemoglobin is used as the protein, examples of useable buffers include, but are not limited to, Clark-Lubs' buffer solution (potassium dihydrogen phosphate + sodium hydroxide), Sorensen's buffer solution (potassium dihydrogen phosphate + sodium tetraborate), Kolthoff's buffer solution (potassium dihydrogen phosphate + sodium tetraborate), Michealis' buffer solution (potassium dihydrogen phosphate + disodium hydrogen phosphate), McIlvain's buffer solution (disodium hydrogen phosphate + citric acid), Britton-Robinson's buffer solution (citric acid + potassium dihydrogen phosphate + boric acid + diethylbarbituric acid + trisodium phosphate), Carmody's buffer solution (boric acid + citric acid + trisodium phosphate), Gomori's buffer solution (2,4,6-trimethylpyridine + Tris-aminomethane + 2-amino-2-methyl-1,3-propanediol + hydrochloric acid), Bates-Bower's buffer solution (Tris-aminomethane + hydrochloric acid), HEPES buffer solution (HEPES + sodium hydroxide + sodium chloride), a broad spectrum buffer solution for measurement of an ultraviolet spectrum (potassium dihydrogen phosphate + citric acid + sodium tetraborate + tris-aminomethane + potassium chloride), and a pH standard solution (potassium dihydrogen phosphate + sodium dihydrogen phosphate).

In the first step of the test method according to the present invention, a solution of a protein, for example, hemoglobin, dissolved in a concentration of 0.001 to 1% by weight, preferably 0.01 to 0.1% by weight, in an aqueous solution prepared by using any of the above-described buffers is placed in a suitable reaction cuvette, and a test substance diluted with the same aqueous solution to a suitable concentration is added to the aqueous protein solution to bring the protein into contact with the test substance through mixing. The contact time and temperature are not limited because optimal conditions vary depending upon the kind and concentration of the protein used. However, the contact time may be usually set to several minutes to several tens of minutes, preferably about 5 min, with the contact temperature being at ambient temperature.

This contact causes the protein to be denatured according to the irritation of a living body by the test substance, which gives rise to a change in the absorption spectrum of the aqueous solution. The degree of the change may be traced by any of the degree of reduction and the degree of increase according to the measuring wavelength. The change in the absorption spectrum may be assayed by reading, with the naked eye, the change in the color tone between before the contact of the protein with the test compound and after the elapse of a given period of time from the initiation of the contact of the protein with the test compound and assaying the degree of change by using a color chart. However, it is preferred to quantitatively determine the absorbance of the solution by means of a spectrophotometer to trace the change in the color tone.

In the evaluation of irritation of a living body based on a test substance, in vivo irritation tests corresponding to intended in vitro irritation tests are conducted to collect data showing various types of irritation (or collecting such known data), the percentage denaturation of proteins is determined according to the test method of the present invention with respect to respective compounds which can be evaluated on the irritation from the above data, and the results are compared with the results on the irritation intensity of the above-described in vivo irritation tests to determined the relationship between the results on the irritation intensity of the in vivo irritation tests and the percentage modification of the proteins determined according to the present invention. The irritation intensity of the test substance can be evaluated from the percentage modification of the protein attributable to the test compound by using the calibration table for the evaluation of the irritation intensity.

According to the present invention, for example, when hemoglobin is used as the protein, the percentage modification of the protein (intensity) has a good correlation with the irritation intensity determined in the ocular irritation method according to the Draize method. The percentage modification of hemoglobin was determined by the following equation (I):

$$
HDR\ (\%) = \frac{\text{Absorbance of control} - \text{Absorbance after modification with test substance}}{\text{Absorbance of control}} \times 100\ (\%)
$$

Substances which can be assayed on the irritation thereof according to the test method of the present invention, that is, test substances, may be substances, materials, or compounds having a possibility that they are directly applied to a living body, and more specific examples thereof include base materials (bases) and auxiliary substances used as cosmetics, medicines, medical materials and fat chemicals. They include pharmacologically active substances, surfactants, fats, fatty acids, humectants, thickeners, buffers, chelating agents, preservatives, ultraviolet absorbers, perfumes and dyes. The test method of the present invention can be conveniently applied to substances having a certain degree of solubility in water among the above-described test substances, especially surfactants and pharmacologically active substances.

The in vitro test of irritation used for the method of the second invention can be any in vitro irritation test so long as it enhances the correlation with the Draize method, in particular, by combination with the first invention. A combination of several tests can further be used. Examples of such tests include the "LIPOSOME", "RBC", "EYTEX", "SIRC", "Hela", and "CAM" methods, as described above. According to the factor analysis of test data based on these methods,

the "EYTEX" method is a typical test using protein denaturation factors different from the first invention, but there is some interaction with membrane disrupting factors, so it is suggested there is some correlation with membrane disrupting factors; the "SIRC" and "Hela" methods are test methods which evaluate cytotoxicity and reveal separate factors, but there is some interaction with membrane disrupting factors, so it is guessed that the "SIRC" and "Hela" tests have some correlation with the membrane disrupting factors as well;

the "CAM" method has the closest relationship to the protein denaturation factor the same as the "EYTEX" method, but also shows some interaction with the protein denaturation factor the same as the first invention and membrane disrupting factors;

the "LIPOSOME" method, from the characteristics of the test, is considered to be a test method reflecting the membrane disrupting factors, which is given support from the analysis results; and

the "RBC" method is a test method having an extremely close relationship to the membrane disrupting factors and almost no interaction with protein denaturation, so is estimated to be a test method for evaluating membrane disruption.

As shown from the analysis results, in the second invention, the other test method selected in addition to the first invention is preferably the "LIPOSOME" or "RBC" method.

According to another aspect of the present invention, there is provided a kit for conducting the above-described test. The kit comprises at least a protein (weighed so that an aqueous solution having a predetermined concentration can be easily prepared) for contact with a test substance or its aqueous solution prepared so that it could be directly brought into contact with the test substance, a buffer, and a reaction cuvette (which is not subjected to any limitation on the configuration and material) for conducting the contact. It may further comprise a microplate having a number of wells for bringing the test specimen and the protein used in the present invention into contact with each other and designed and prepared so that a plurality of test samples can be simultaneously measured, and other equipment for experiments, for example, a pipette, a beaker, an agitator, and a manual. They may be packaged separately from each other or in one unit.

In the method according to the present invention, the change in the spectrum of the protein may be determined by means of a spectrophotometer usually provided in general laboratories. Alternatively, use may be made of a method wherein the change in the spectrum on the above-described number of wells are determined at one time. The obtained data are applied to a system designed in such a manner that the data can be subjected to numerical analysis by means of a computer.

In the second invention, when the "LIPOSOME" method is used as the method other than the first invention, the kit can comprise reagents described in the above-described Japanese Unexamined Patent Publication No. 63-228060, specifically, a marker-containing liposome, for example, a reagent prepared from at least one reagent selected from the group consisting of a naturally occurring phospholipid, a synthetic phospholipid, a naturally occurring glycolipid, a synthetic glycolipid, a quaternary ammonium salt, a higher alcohol, and a mixture thereof, with at least one neutral fat.

Alternatively, when the "RBC" method is selected as the other method, the kit can comprise cell preparations which are used for carrying out a method as described in Wolfgang et al., Molecular Toxicology, 1, pp. 525-536 (1987).

## EXAMPLES

The present invention will now be described in more detail with reference to the following examples, though it is not limited to these examples.

### Example 1: Correlation of Hemoglobin Denaturalizing Ratio with Draize Method

Michaelis' buffer solution (potassium dihydrogen phosphate + disodium hydrogen phosphate) was adjusted to a pH value of 6.80 and a concentration of 50 mmol. A 0.05% (w/w) hemoglobin (manufactured by Wako Pure Chemical Industries, Ltd.) was dissolved in the adjusted buffer solution to prepare a hemoglobin buffer solution. Separately, a 0.5% aqueous solution of a test substance was prepared. 1.2 ml of the hemoglobin buffer solution was placed in a test tube, and 1.2 ml of the aqueous test substance solution was added and mixed therewith to prepare a homogeneous mixture which was then transferred to an absorbance measuring cell and subjected to measurement of the absorbance at 405 nm (a time interval of about 5 min was provided from the mixing to the measurement for the purpose of stably obtaining data).

In order to measure the background absorbance, 1.2 ml of Michael' buffer solution was placed in a test tube, and 1.2 ml of the aqueous test substance solution was added and mixed therewith to prepare a homogeneous mixture which was then transferred to an absorbance measuring cell and subjected to measurement of the absorbance at 405 nm (a time interval of about 5 min was provided from the mixing to the measurement for the purpose of stably obtaining data). The background absorbance was subtracted from the absorbance of the mixed solution comprising the hemoglobin and the test substance to determine the absorbance of the test substance.

1.2 ml of a hemoglobin buffer solution was placed in a test tube, 1.2 ml of deionized water was added as a control and mixed with the hemoglobin buffer solution to prepare a homogenous mixture which was then transferred to an absorbance measuring cell and subjected to measurement of the absorbance at 405 nm (a time interval of about 5 min was provided from the mixing to the measurement for the purpose of stably obtaining data). In order to measure the background absorbance of the control, 1.2 ml of the hemoglobin buffer solution was placed in a test tube, and 1.2 ml of deionized water was added and mixed therewith to prepare a homogeneous mixture which was then transferred to an absorbance measuring cell and subjected to measurement of the absorbance at 405 nm (a time interval of about 5 min was provided from the mixing to the measurement for the purpose of stably obtaining data). The absorbance measured as the background of the control was subtracted from the absorbance of the control to determine the absorbance of the test substance. The hemoglobin denaturalizing ratio (HDR) was calculated from these measurements according to the above-described equation (I).

The same test substance as that used above was subjected to a test according to the Draize method.

The results are given in Table 1.

Table 1

| Name of test substance | Conc. of test substance | HDR(%) | Ocular irritation | Primary ocular irritation (Draize score) |
|---|---|---|---|---|
| SDS* | 0.5% | 32.68 | 35.1 | 6.9 |
| Cetyl pyridinium chloride | 0.5% | 48.85 | 70 | 4.0 |
| Octyl phenyl ether | 0.5% | 1.25** | 8.7 | 0.2 |

Note) *: SDS represents sodium dodecylsulfate
**: A HDR value of 1.25% corresponds to the ocular irritation in terms of Draize score in $\gamma = 0.97$ and to the primary skin irritation in terms of Draize score in $\gamma = 0.71$.

As is apparent from Table 1, the coefficient of correlation of the hemoglobin denaturalizing ratio with ocular irritation was 0.97, and the coefficient of correlation of the hemoglobin denaturalizing ratio with skin irritation was 0.71. That is, a very good correspondence could be obtained. Further, when a comparison was conducted with the concentration being identical, it was found that test substances having strong ocular irritation and skin irritation had a high hemoglobin denaturalizing ratio while test substances having weak ocular irritation and skin irritation had a low hemoglobin denaturalizing ratio.

Example 2(comparative): Correlation of Test for Hemolysis of Erythrocytes with Draize Method

(1) Preparation of Suspension Containing Erythrocytes

Wistar line rats (300 to 350g) were anesthetized by ether, and blood was drawn through a heparinized cannula from the aorta abdominalis of the same. The erythrocytes were isolated by centrifugation of obtained blood samples at 3000 r.p.m. for 10 minutes. The resulting erythrocytes were washed with physiological saline, and dispersed in an isotonic phosphate buffer at 7.4 of pH, to 2% by the weight, to prepare a suspension containing erythrocytes.

(2) Determination of the Concentration of the Test Substance to Cause Hemolysis

A sample containing test substance was prepared with an isotonic phosphate buffer at 7.4 of pH. Using the sample the range from the concentration resulting in complete hemolysis of the erythrocytes to the concentration with no hemolysis at all was determined in advance by preliminary tests. The range was divided into 10 steps of concentration and the main test was performed. The concentration of hemolysis 2 ml portions of the suspension erythrocytes in each test tube, was determined by preparing two test tubes for each concentration of the test substance, taking quickly adding the same amount of the test substance solution using a syringe, then allowing a reaction at 32°C for 30 minutes to cause hemolysis of the erythrocytes. After 30 minutes, the result was centrifuged at 1500 r.p.m. for 10 minutes to precipitate the unhemolyzed erythrocytes, the supernatant was diluted five-fold with a phosphate buffer, then the hemoglobin content was determined using a wavelength of 545 nm. Note that as a blank, use was made of a solution obtained by adding the phosphate buffer instead of the test substance and processing it in the same way. The concentrations of the test substances are shown in Table 2. In the same giving complete hemolysis way as in Table 1, the results of the Draize method are simultaneously shown.

## Table 2

| Test substance | Conc. for hemolysis (μg/ml) | Draize score | |
| | | Ocular irritation | Primary skin irritation |
| --- | --- | --- | --- |
| SDS | 70 | 35.1 | 6.9 |
| Cetyl pyridinium chloride | 31 | 70 | 4.0 |
| Octyl phenyl ether | 160 | 8.7 | 0.2 |

Note) *RBC method corresponds to $\gamma = -0.95$ at the ocular irritation of Draize score, and $\gamma = -0.73$ at the primary skin irritation, respectively.

Example 3

A commercially available pH standard solution (manufactured by Wako Pure Chemical Industries, Ltd.) was prepared, and 0.05% (w/w) hemoglobin (a guaranteed reagent manufactured by Wako Pure Chemical Industries, Ltd.) was dissolved in the pH standard solution to prepare a hemoglobin buffer solution. Separately, a 2.0% aqueous solution of each test sample was prepared.

The following samples were prepared by using a transparent resin plate having $8 \times 12$ (= 96) compartments.

① Test substance: 11 concentration levels × n4 (hemoglobin buffer)

② Test substance: 11 concentration levels × n4 (buffer)

③ Ion-exchanged water: control × n4 (hemoglobin buffer)

④ Ion-exchanged water: control × n4 (buffer)

In the samples, ② is a control for ①, and ④ is a control for ③. The 11 control levels were prepared by serial two-fold dilution with the initial concentration being 2%.

The samples were allowed to stand for 5 minutes, shaken for 20 seconds, and subjected to measurement of the absorbance at 415 nm (which is a value for an absorption peak of hemoglobin described in a literature). The measurement was automatically conducted on 96 wells by means of EIA-READER. The data of the absorbance were processed by a computer to calculate the hemoglobin denaturation ratio on 11 concentration levels. The denaturation ratio was calculated in terms of the average value of n4. The results are summarized in Table 3.

Table 3

| Conc. of surfactant (%) | 2.000 | 1.000 | 0.500 | 0.250 | 0.125 | 0.063 | 0.031 |
|---|---|---|---|---|---|---|---|
| Cetyl pyridinium chloride | 55.21 | 48.21 | 48.85 | 49.15 | 50.50 | 50.45 | 45.91 |
| SDS* | 41.22 | 37.41 | 32.68 | 30.57 | 15.03 | 5.28 | 2.52 |
| Octyl phenyl ether | 0.00 | 7.03 | 1.26 | 1.20 | 0.00 | 0.00 | 0.00 |

Table 3 (continue)

| Conc. of surfactant (%) | 0.016 | 0.008 | 0.004 | 0.002 | Estimated value | Actual evaluation | Correlation coefficient |
|---|---|---|---|---|---|---|---|
| Cetyl chloride | 30.11 | 10.57 | 1.35 | 0.00 | 66.544 | 70.000 | |
| SDS* | 0.00 | 0.00 | 0.00 | 0.00 | 43.331 | 35.100 | 0.994 |
| Octyl phenyl ether | 0.00 | 0.00 | 0.00 | 0.00 | 17.610 | 3.700 | |

Note) *: SDS represents sodium dodecylsulfate

The results thus obtained were subjected to regression using the following associative equation to obtain the above-described correlation coefficient.

17.61 + 0.624 1% HDR + 0.481 0.016% HDR

Consequently, a very good correspondence was observed even with evaluation by a plurality of concentrations.

# EP 0 600 087 A1

Example 4

Method 1: The procedure as described in Example 1 was repeated.
Method 2: The procedure as described in Example 2 was repeated.

The results obtained from the two methods were subjected to regression using the <u>associative equation</u> below:

| | |
|---|---|
| 8.799 - 0.0066 | * RBC |
| + 1.33468 | * 2% HDR |
| - 0.6398 | * 0.25% HDR |
| + 0.65449 | * 0.063% HDR. |

The results are summarized in Table 4.

Table 4

| Test substance | Calculated value | Found value |
|---|---|---|
| SDS | 70.5078 | 70 |
| Cetyl pyridinium chloride | 33.9028 | 35 |
| Octyl phenyl ether | 6.9685 | 8.7 |
| * Correlation coefficient between the calculated values and the found values is 0.9999. | | |

Here, the calculated values are estimated values based on the Draize method using the above-described associative equation, and the found values are measurements according to the Draize method.

INDUSTRIAL APPLICABILITY

According to the present invention, there are provided in vitro test methods for irritation of the body by various test substances, in particular, ones which exhibit a high correlation with the results of measurement if irritation of the living body derived from the Draize method. These methods can be used for safety testing of a variety of products in the industry in cosmetic and pharmaceutical manufacturing industries.

**Claims**

1. A <u>in vitro</u> test method of irritation of a living body using a test substance, comprising the steps of:
   (A) bringing a test substance into contact with a metalloprotein or a modified protein having an absorption spectrum in a visible region in an aqueous solution;
   (B) determining the change in the absorption spectrum attributable to said contact; and
   (C) evaluating the change in said spectrum as an indicator of the irritation of the living body based on the test substance.

2. A method according to claim 1, wherein said protein is a hemoglobin.

3. An <u>in vitro</u> test method of irritation of a living body using a test substance, comprising the steps of:
   a) a step of separately conducting the test method according to claim 1 and at least one other <u>in vitro</u> irritation test method which has a good correlation with the Draize eye irritation test for the identical test substance and
   b) a step of collectively evaluating the results based on step a).

4. A method according to claim 3, wherein said <u>in vitro</u> test method for irritation is selected from the group consisting of "LIPOSOME" method using disruption of liposome as an indicator; an "RBC" method using hemolysis of erythrocytes; an "EYTEX" method using a tendency of clouding based on denaturation of protein such as albumin as an indicator; an "SIRC" method which is a cytotoxic test using cultured cells derived from rabbit cornea; and a "HeLa" method which is a cytotoxic test using cultured cells derived human cervical carcinoma.

11

5. A method according to claim 4, wherein said test method is the "LIPOSOME" or "RBC" method.

6. A kit for an in vitro test method of irritation of a living body using a test substance, comprising:
    a) a device for bringing a test substance into contact with a metalloprotein or a modified protein having an absorption spectrum in a visible region in an aqueous solution and said protein and
    b) means for evaluating the change in the absorption spectrum caused in said device as an indicator of the irritation of the living body based on the test substance.

7. A kit according to claim 6, which further includes test materials required to conduct the other in vitro irritation test method according to claim 4.

8. A kit according to claim 7, wherein said test material required to conduct the other in vitro test method of irritation is a liposome containing a marker.

# INTERNATIONAL SEARCH REPORT

International Application No PCT/JP92/01045

**I. CLASSIFICATION OF SUBJECT MATTER** (if several classification symbols apply, indicate all) [6]

According to International Patent Classification (IPC) or to both National Classification and IPC

Int. Cl$^5$  G01N33/15

**II. FIELDS SEARCHED**

| | Minimum Documentation Searched [7] |
|---|---|
| Classification System | Classification Symbols |
| IPC | G01N33/15 |

Documentation Searched other than Minimum Documentation
to the Extent that such Documents are Included in the Fields Searched [8]

| | |
|---|---|
| Jitsuyo Shinan Koho | 1926 – 1992 |
| Kokai Jitsuyo Shinan Koho | 1971 – 1992 |

**III. DOCUMENTS CONSIDERED TO BE RELEVANT [9]**

| Category [*] | Citation of Document, [11] with indication, where appropriate, of the relevant passages [12] | Relevant to Claim No. [13] |
|---|---|---|
| X,Y | JP, A, 61-502074 (Preventive Diagnostics Corp.), September 18, 1986 (18. 09. 86), & US, A, 4613574 & EP, A, 180626 | 1, 3-8 |
| Y | JP, A, 63-228060 (Shiseido Co., Ltd.), September 22, 1988 (22. 09. 88), (Family: none) | 3-5, 7, 8 |
| Y | J. Soc. Cosmet. Chem. Japan, Vol. 123, No. 4, (199), Yuuko Okamoto et al., "Study on Substitution for Eye Irritative Test" — Comparison and Examination of Methods using Hemolysis and Culture Cells — p. 272-279 | 4-5 |
| Y | Toxic. in vitro, Vol. 4, Nos. 4, 5, (1990), S. C. Johnson et al., "AN IN VITRO METHOD FOR ESTIMATING OCULAR IRRITATION", p. 332-335 | 4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 2, 1992 (02. 11. 92) | November 17, 1992 (17. 11. 92) |
| International Searching Authority | Signature of Authorized Officer |
| Japanese Patent Office | |

Form PCT/ISA/210 (second sheet) (January 1985)